# EUROPEAN PATENT SPECIFICATION

(11) **EP 1 001 770 B1**
(45) Date of publication and mention of the grant of the patent: **24.08.2005**
(21) Application number: 98930109.8
(22) Date of filing: 10.06.1998
(51) Int. Cl.: A61K 31/44, A61K 31/4436, A61N 5/06, A61P 17/06

(54) **TAZAROTENE AND UVB PHOTOTHERAPY TREATMENT FOR PSORIASIS**
TAZAROTEN UND UVB-PHOTOTHERAPIE ZUR BEHANDLUNG VON PSORIASIS
TRAITEMENT DU PSORIASIS A BASE DE TAZAROTENE ET DE PHOTOTHERAPIE AUX RAYONS UVB

(30) Priority: 11.06.1997 US 49385 P
(43) Date of publication of application: 24.05.2000
(73) Proprietor: ALLERGAN, INC., Irvine, CA 92612 (US)
(72) Inventor: SEFTON, John, Trabuco Canyon, CA 92678 (US); LEW-KAYA, Deborah, A., Newport Coast, CA 92657 (US)
(74) Representative: HOFFMANN EITLE
(86) International application number: PCT/US1998/011989
(87) International publication number: WO 1998/056375

(56) References cited:
- WO-A-95/33489
- MARK LEBWOHL: "Future psoriasis therapy" DERMATOLOGIC CLINICS, vol. 13, no. 4, 1995, pages 915-923, XP002080624
- CARL W. EHMANN ET AL.: "International studies of the efficacy of etretinate in the treatment of psoriasis" JOURNAL OF THE AMERICAN ACADEMY OF DERMATOLOGY, vol. 6, no. 4 PT 2 suppl., 1982, pages 692-696, XP002080625

## Description

### BACKGROUND OF THE INVENTION

### 1. Field of the Invention

The present invention relates to a medicament for treating psoriasis in humans with Tazarotene, preferably a gel comprising 0.1%, Tazarotene by weight, and phototherapy, i.e. UVB phototherapy. The Tazarotene gel may be topically administered followed by UVB phototherapy at the rate of three treatments per week.

### 2. Description of Related Art

Psoriasis is a chronic skin disease which is known to be difficult to treat. Psoriasis is characterized by discrete and confluent, reddish, silvery-scaled maculopapules. These psoriatic lesions occur most often on the elbows, knees, trunk and scalp. Current treatment for psoriasis include the use of agents such as anthralin (dihydroxyanthralin), azarabine, colchicine, fluorouracil, methorexate, methoxsalen (8-methoxypsoralen), resorcinol, retinoids (for example, retinoic acid), corticosteroids (for example, clobetasol propionate, trimcinolone acetonicde and the like), cyclosporin, iodochlorhydroxyquin, salicyclic acid, vitamin D, dapsone, somoatostatin, sulfur, tars and zinc oxide. Ultra-violet light treatment, alone or in combination with other agents such as psoralen (i.e., PUVA treatment), is also used to treat psoriasis.

It is also known to treat skin diseases such as psoriasis, seborrheic dermatitis and eczema with sunlight baths or ultraviolet (UV) baths with lesions painted with a solution of coal tar, anthralin or/psoralens (See U.S. Patent Numbers 5,681,593 and 5,122,514). Other references suggesting photo therapeutic treatment of proliferative skin diseases, such as psoriasis, include U.S. Patent 4,753,958, wherein UV light is utilized in combination with phototoxic derivatives of hematoporphyrin. See also U.S. Patent 5,556,612 wherein optical radiation is combined with a topical sunscreen to provide preferential photoprotection in phototherapy, photochemotherapy and photodynamic therapy.

Various devices for providing UV radiation for treating skin diseases such as psoriasis are disclosed in U.S. Patents 4,177,384; 4,287,554; 4,370,595 and 4,560,883.

The safety, efficacy, and patient acceptability of tazarotene 0. 1 % and 0.05 % gels when used in the treatment of psoriasis were evaluated in two phase 3 clinical research studies. Based on promising results from these studies, Allergan, Inc. filed a new drug application for tazarotene in June, 1995 which was approved by the Food and Drug Administration (FDA).

### BRIEF SUMMARY OF THE INVENTION

The present invention provides a medicament for treating psoriasis in a human subject by topically applying to the psoriasis of said subject an effective amount of tazarotene and an effective amount of UVB radiation.

Preferably, in the medicament of the invention, tazarotene is to be applied as a 0.05% or 0.1% by weight, gel.

In the preferred medicament of the invention, tazarotene is to be administered once daily in the evening.

Preferably, the UVB phototherapy is administered by means of a Houva 11 UVB Box, which is available from National Biological Corporation.

### BRIEF DESCRIPTION OF THE DRAWING FIGURES

Figure 1 compares the reduction in plaque achieved by the method of the present invention, i.e. the combination of treatment with tazarotene and UVB radiation, and UVB radiation treatment, alone.
Figure 2 compares the reduction in scaling achieved by the method of the present invention, i.e. the combination of treatment with tazarotene and UVB radiation, and UVB radiation treatment, alone.
Figure 3 compares the reduction in erythema achieved by the method of the present invention, i.e. the combination of treatment with tazarotene and UVB radiation, and UVB radiation treatment, alone.
Figure 4 compares the treatment success rate achieved by the method of the present invention and UVB radiation treatment, alone.
Figure 5 compares the reduction in plaque achieved by the method of the present invention and UVB radiation treatment, alone, and UVB radiation treatment in combination with placebo, i.e. the tazarotene vehicle.
Figure 6 compares the reduction in scaling achieved by the method of the present invention and UVB radiation treatment, alone, and UVB radiation treatment in combination with placebo, i.e. the tazarotene vehicle.
Figure 7 compares the reduction in erythema achieved by the method of the present invention and UVB radiation treatment, alone, and UVB radiation treatment in combination with placebo, i.e. the tazarotene vehicle.
Figure 8 compares treatment success rate achieved by the method of the present invention and UVB radiation treatment, alone, and UVB radiation treatment in combination with placebo, i.e. the tazarotene vehicle.

### DETAILED DESCRIPTION OF THE INVENTION

The preferred tazarotene gel for use in the method of this invention is disclosed in U.S. Patent Application Serial No. 623,184, which is entitled "Stable Gel Formulation for Topical Treatment of Skin Conditions", which was filed on March 28, 1996, in the name of Prakash Charu. (Said preferred gel is also described in published PCT Application No. PCT/US95/07338 or WO 95/33489.)

A method for preparation of a gel formulation of tazarotene for topical treatment of psoriasis comprises the steps of:
1) mixing purified water, edetate disodium,
   ascorbic acid and Carbomer 934P i.e. a polymer of acrylic acid crosslinked with allyl ethers of sucrose, until the carbomer is dispersed to form a part I;
2) mixing purified water, Poloxamer 407, i.e. α-Hydro-ω-hydroxypoly (oxyethylene)ₐ-poly(oxopropylene)_{b}-poly(oxyethylene)ₐ block polymer, wherein the average value of a is 101 and the average value of b is 56, to form a part II;
3) adding part II to part I and homogenizing part I and Part II;
4) mixing polyethylene glycol, Polysorbate 40, i.e. polyoxyethylene 20 sorbitan monopalmitate, hexylene glycol, butylated hydroxytoluene and butylated hydroxyanisole and heating to dissolve same;
5) cooling the mixture of step 4) to room temperature and adding benzyl alcohol and tazarotene thereto to form a part III;
6) mixing purified water and tromethamine to form part IV;
7) adding part III to parts I and II while stirring and thereafter adding part IV with mixing until homogeneous.

Both the 0.05% and the 0.1%, by weight, tazarotene gel formulations are available from Allergan, Inc. of Irvine, CA under the brand name Tazorac®.

### DESCRIPTION OF THE PREFERRED EMBODIMENT

The hypothesis for the study described below was that Tazarotene plus UVB, as compared to UVB plus vehicle or UVB alone would work faster, have higher success rate, require less UVB exposure and be well tolerated. The Study objective was to assess safety and efficacy of pre-treatment with Tazarotene 0.1% gel and UVB phototherapy in comparison with the Tazarotene vehicle (placebo) and UVB phototherapy and UVB phototherapy, alone, for 10 weeks. During UVB phototherapy, topical agents were applied three times weekly to the target lesion by the phototherapy nurse. The study was a multicenter, investigator-masked study including 60 subjects and 120 target lesions. During the two-week pre-UVB phototherapy period the patients were randomly assigned to receive two of the following treatments: Tazarotene 0.1% gel once a day; vehicle gel once a day; or no treatment. The topical agents were applied once daily. Prior to UVB therapy, the minimal erythemal dose (MED) was assessed using a range of 20 mJoules/cm² up to 100 mJoules/cm². Initial starting UVB dose is 25% of MED. If MED is immeasurable, starting dose is 25 mJoules/cm². During the ten-week UVB phototherapy period the patients were treated with UVB therapy three times a week and topical therapy three times weekly (0.1% Tazarotene gel, vehicle gel, or no treatment) administered by the phototherapy nurse after UVB exposure. Study population was as follows: male or non-reproductive female; 18 years or older; stable plaque psoriasis; two bilaterally symmetrical plaques accessible to phototherapy; not exceeding 50% of TBS; baseline plaque elevation of at least four (moderate, in 0-8 scoring system); no oral retinoid within two months; no excessive vitamin A intake; no oral psoriatic therapy (other than retinoid) or PUVA within one month; no UVB within two weeks; no significant systemic disease or immunosuppression. The UVB exposure was increased until either improvement in psoriasis was noted or MED was reached. If one target lesion reached MED earlier than the other, a photo-opaque covering was applied to the former so the latter site could still be treated with a higher dose of UVB irradiation. If both target lesions cleared, UVB was discontinued. If one target lesion cleared and the other shows ≥ 75% improvement, UVB was discontinued.
The following evaluation criteria was used:
Efficacy Variable
   - Plaque elevation, scaling, erythema:
      Grading Scale
      0 = none, 2= mild, 4=moderate, 6=severe, 8=very severe (with whole-point increments (1, 3, 5, 7) serving as mid-points)
   - Global response to treatment
   - Total cumulative UVB exposure (mJoules/cm²)

### Global Response to Treatment

Treatment success is defined as moderate response or better.

| | | |
|---|---|---|
| 0 | Completely cleared | |
| 1 | Almost cleared | about 90% improvement |
| 2 | Marked response | about 70% improvement |
| 3 | Moderate response | about 50% improvement |
| 4 | Slight Response | about 25% improvement |
| 5 | Condition Unchanged | |
| 6 | Condition Worsened | |

10 patients were enrolled in the study. The patients that we used in the study had the following demographic constitution:

| Ten male patients | Mean | Range |
|---|---|---|
| Age (years) | 48 | 27-67 |
| Weight (kg) | 91 | 61-134 |
| Height (cm) | 180 | 173-193 |

### Race

| | |
|---|---|
| Caucasian | 8 |
| Black | 1 |
| Hispanic | 1 |

Nine patients completed the study. One was discontinued due to UVB burn, not as a result of the topical-treatment.

The results are reported in the following figures.

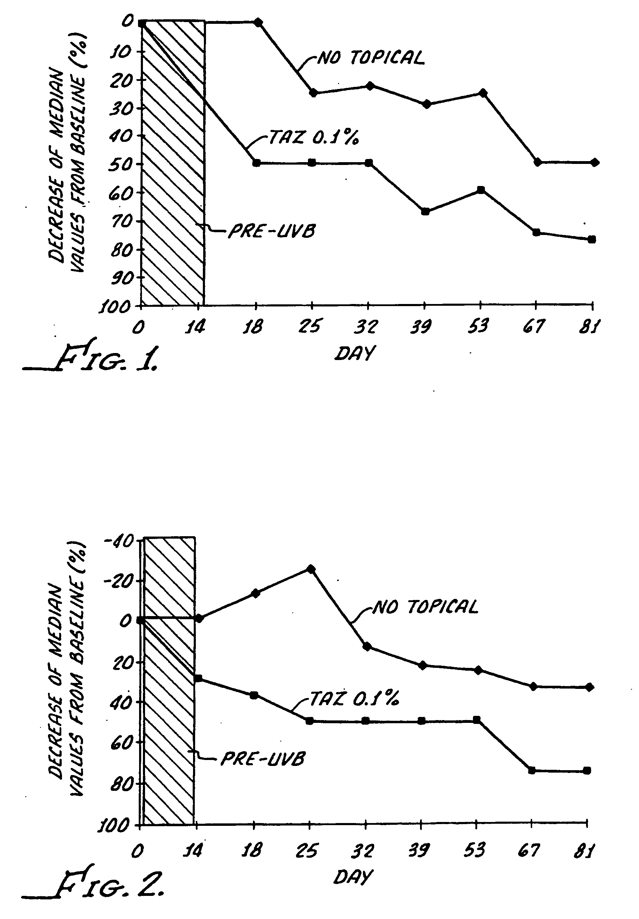

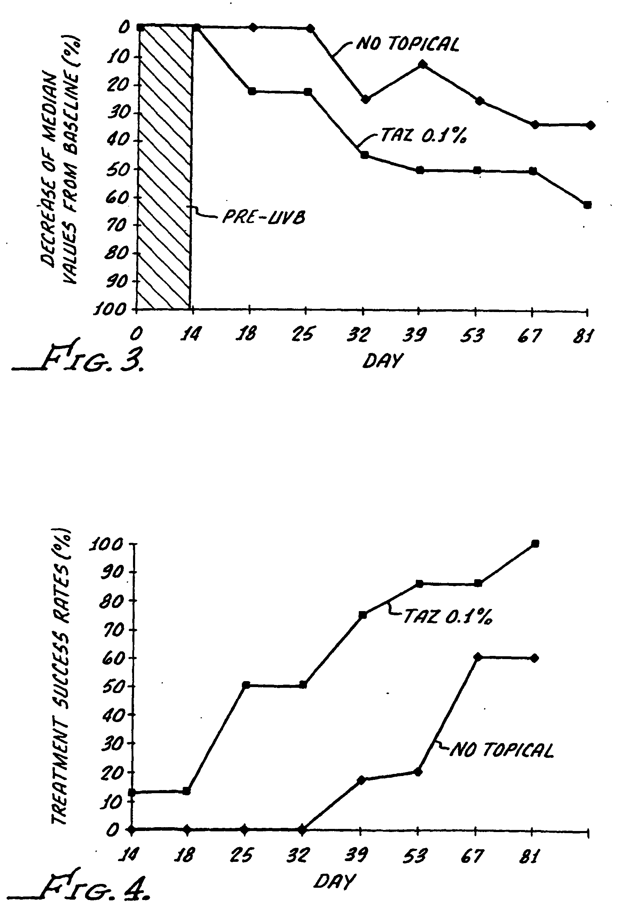

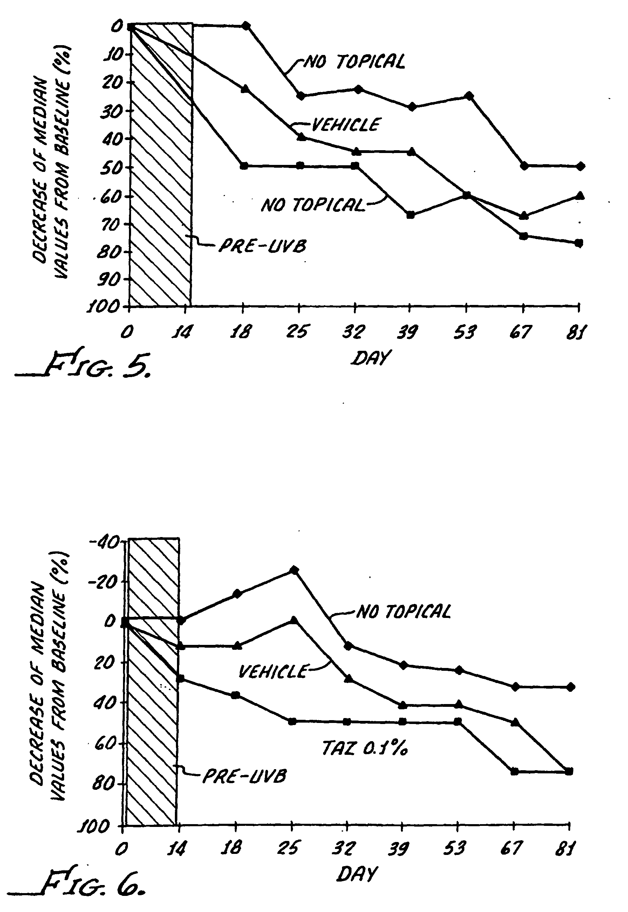

The reduction in plaque, scaling and erythema are reported in Figures 1 through 3, respectively. Note, in each case Tazarotene in combination with treatment with UVB was more effective than UVB treatment alone.

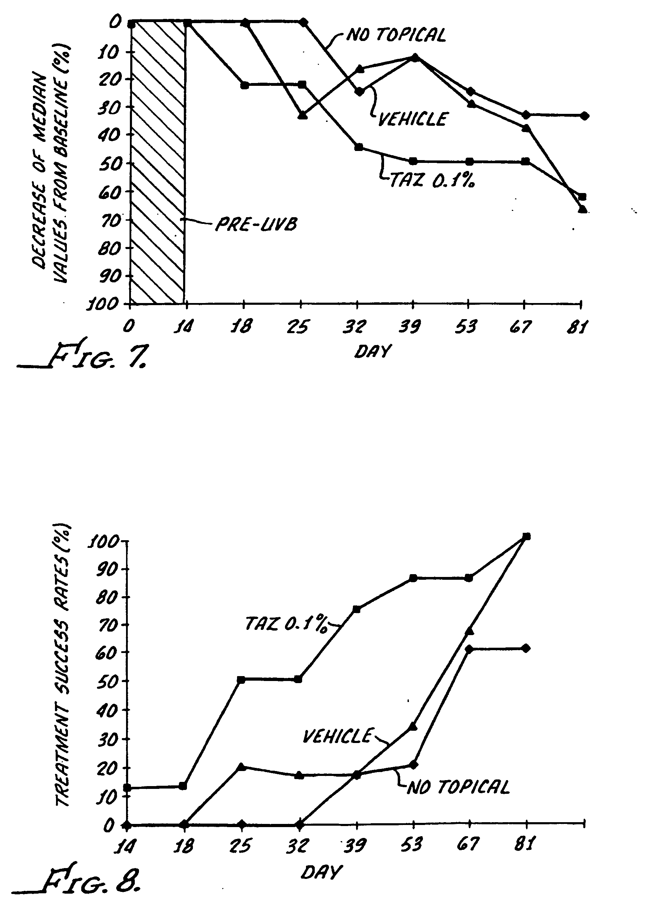

As shown in Figure 4, the treatment success rate with Tazarotene and UVB phototherapy was 100% by day 81. At the same time, UVB phototherapy, alone, showed only about a 60% success rate.

In Figures 5 through 8, the reductions in plaque, scaling and erythema, respectively, are greater with Tazarotene and UVB treatment, than with either UVB, alone, or UVB with vehicle (placebo).

In Figure 8 both Tazarotene and UVB treatment and vehicle and UVB treatment show a 100% success rate at 81 days. However, the treatment with Tazarotene and UVB achieves a successful treatment more quickly than vehicle and UVB treatment. For example, at day 53, about 90% success is achieved with Tazarotene and UVB, while about 30% success is achieved with vehicle and UVB.

The following adverse events were reported during the study:

| | Tazarotene | Vehicle |
|---|---|---|
| Perilesional irritation | 2 | 0 |
| Perilesional tenderness | 0 | 1 |
| Lesional irritation | 0 | 1 |
| Burning sensation | 1 | 0 |
| Itching | 1 | 0 |

### All were mild in severity

In conclusion, treating psoriasis in humans with a combination of Tazarotene and UVB phototherapy is more effective than a combination of the Tazarotene vehicle and UVB phototherapy or UVB phototherapy, alone.

The following conclusions were reached in this study. Reductions in plaque elevation and scaling were generally greater for plaques treated with UVB plus Tazarotene 0.1% gel. Less time was required to reach treatment success for plaques treated with UVB plus Tazarotene 0.1% gel. Combination therapy was well tolerated and no significant UVB-Tazarotene phototoxicity noted.

## Claims

1. Use of an effective amount of tazarotene for the manufacture of a medicament for the topical treatment of psoriasis in combination with UVB radiation.

2. Use according to claim 1 wherein the medicament is a 0.1% tazarotene gel.

3. Use according to claim 1 wherein the UVB radiation is to be generated by means of a Houva 11 UV box.

4. Use according to claim 1 wherein the tazarotene is to be applied three times weekly followed by UVB radiation.

5. Use according to claim 1 wherein the dose of UVB radiation is 25% of MED.

6. Use according to claim 1 wherein the dose of UVB radiation is within the range of 20 to 100 mJoule/cm².

7. Use according to claim 1 wherein the UVB radiation is to be applied for two weeks followed by applying tazarotene and UVB radiation for 10 weeks.

## Patentansprüche

1. Verwendung einer wirksamen Menge Tazaroten zur Herstellung eines Medikaments für die topische Behandlung von Psoriasis in Kombination mit UVB-Strahlung.

2. Verwendung gemäss Anspruch 1, worin das Medikament ein 0,1 %-iges Tazarotengel ist.

3. Verwendung gemäss Anspruch 1, worin die UVB-Strahlung mittels einer Houva 11 UV-Box erzeugt wird.

4. Verwendung gemäss Anspruch 1, worin das Tazaroten dreimal wöchentlich, gefolgt von UVB-Bestrahlung, angewandt wird.

5. Verwendung gemäss Anspruch 1, worin die UVB-Strahlungsdosis 25 % MED beträgt.

6. Verwendung gemäss Anspruch 1, worin die UVB-Strahlungsdosis im Bereich von 20-100 mJ/cm² liegt.

7. Verwendung gemäss Anspruch 1, worin die UVB-Strahlung für 2 Wochen aufgebracht wird, gefolgt von der Anwendung von Tazaroten und UVB-Bestrahlung für 10 Wochen.

## Revendications

1. Utilisation d'une quantité efficace de tazarotène pour la fabrication d'un médicament destiné au traitement topique du psoriasis en combinaison avec une radiation UVB.

2. Utilisation selon la revendication 1, dans laquelle le médicament est un gel de tazarotène à 0,1%

3. Utilisation selon la revendication 1, dans laquelle la radiation UVB doit être générée au moyen d'une boîte UV Houva 11.

4. Utilisation selon la revendication 1, dans laquelle le tazarotène doit être appliqué trois fois par semaine suivi d'une radiation UVB.

5. Utilisation selon la revendication 1, dans laquelle la dose de radiation UVB est de 25% de MED.

6. Utilisation selon la revendication 1, dans laquelle la dose de radiation UVB se situe dans la plage de 20 à 100 mJoule/cm².

7. Utilisation selon la revendication 1, dans laquelle la radiation UVB doit être mise en oeuvre pendant deux semaines suivie d'une application de tazarotène et d'une radiation UVB pendant 10 semaines.
